# EUROPEAN PATENT SPECIFICATION

(11) **EP 3 665 479 B1**
(45) Date of publication and mention of the grant of the patent: **14.09.2022**
(21) Application number: 18755763.2
(22) Date of filing: 09.08.2018
(51) Int. Cl.: G01N 33/49, G01N 27/333

(54) **APPARATUS FOR ACCURATE SENSING OF PHYSIOLOGICAL SUBSTANCE IN BLOOD**
VORRICHTUNG FÜR GENAUE MESSUNG EINER PHYSIOLOGISCHEN SUBSTANZ IM BLUT
DISPOSITIF POUR DÉTECTION PRÉCISE DE SUBSTANCE PHYSIOLOGIQUE DANS LE SANG

(30) Priority: 09.08.2017 DE 102017118147
(43) Date of publication of application: 17.06.2020
(73) Proprietor: Joanneum Research Forschungsgesellschaft mbH, 8010 Graz (AT)
(72) Inventor: PEKEC, Bruna, 8010 Graz (AT); HAJNSEK, Martin, 8010 Graz (AT)
(74) Representative: Dilg, Haeusler, Schindelmann Patentanwaltsgesellschaft mbH
(86) International application number: PCT/EP2018/071662
(87) International publication number: WO 2019/030338

(56) References cited:
- EP-A1- 1 482 307
- EP-A1- 2 050 824
- WO-A1-95/10044
- WO-A1-2016/141337
- US-A1- 2014 262 831
- US-A1- 2017 020 424
- LIYING JIANG ET AL: "Disposable Biosensor for Hemoglobin Determination in Whole Blood", NANO/MICRO ENGINEERED AND MOLECULAR SYSTEMS, 2006. NEMS '06. 1ST IEEE INTERNATIONAL CONFERENCE ON, IEEE, PI, 1 January 2006 (2006-01-01), pages 204-208, XP031063830, ISBN: 978-1-4244-0139-0
- KOBOS R K ET AL: "Electrochemical determination of hemoglobin, hematocrit, and hemolysis", CLINICAL CHEMISTRY, AMERICAN ASSOCIATION FOR CLINICAL CHEMISTRY, WASHINGTON, DC, vol. 33, no. 1, 1 January 1987 (1987-01-01), pages 153-158, XP002725061, ISSN: 0009-9147

## Description

The invention relates to a sensor arrangement. Moreover, the invention relates to a monitoring system.

A potassium test is used to measure the amount of potassium in a patient's blood. Potassium is an electrolyte that is essential for proper muscle and nerve function. Electrolytes become ions when they are in a solution, and they conduct electricity. Human cells and organs require electrolytes to function normally. Even minor increases or decreases in the amount of potassium in a patient's blood can result in serious health problems.

A potassium test can be performed as a blood test. A blood sample drawn by a patient's healthcare provider will be sent to a laboratory for analysis, and a doctor will review the results.

However, conventional potassium tests and other blood tests frequently suffer from poor accuracy and the need to involve medical experts.

US 2014/0262831 A1 discloses an electrochemical sensor system for detecting hemolysis in a whole blood sample, wherein the hemolysis is detected indirectly by electrochemically determining the depletion of enzymatically generated hydrogen peroxide in the presence of Hb(Fe²⁺). To this end, the electrochemical sensor comprises a membrane comprising an oxidoreductase enzyme capable of generating hydrogen peroxide. Another method of detecting hemolysis in a whole blood sample is described in WO 95/10044, wherein a whole-blood sample comprising intact red blood cells is contacted with a dry separation material having physical characteristics effective to separate a fraction, which contains extracellular hemoglobin that may be present in said sample, from the intact red blood cells, the sample is allowed to remain in contact with the material for a period of time sufficient to effect separation and the presence of extracellular hemoglobin is detected in the fraction. EP 1 482 307 A1 discloses a biosensor comprising an insulating substrate, an electrode system, a reaction layer containing oxidation reduction enzyme and electronic mediator, a reagent supply passage having an inlet and ventilation holes and containing the electrode systems and the reaction layer, a reagent supply part, and a first filter between the supply part and the passages. Jiang et al., Conference on Nano/Micro Engineered and Molecular Systems 2006 discloses a disposable biosensor for hemoglobin determination in whole blood based on amperometric detection technology. EP 2 050 824 A1 discloses an electrochemical method and a test strip for detecting hemoglobin in a specimen.

It is an object of the invention to provide a blood test with high accuracy and low effort.

In order to achieve the object defined above, a sensor arrangement in accordance with claim 1, and a monitoring system in accordance with claim 10 are provided.

According to an exemplary embodiment of the invention, a sensor arrangement for detecting information indicative of a physiological substance (for instance detecting an amount or a concentration of the physiological substance) in blood of a physiological subject is provided, wherein the sensor arrangement comprises a first sensor structure configured for electrochemically sensing the physiological substance in at least part of the blood, wherein detecting the physiological substance is influenceable by hemolysis of the blood, a second sensor structure configured for electrochemically sensing a parameter in at least part of the blood, which parameter is indicative of hemolysis of the blood, wherein the second sensor structure is configured for electrochemically sensing hemolysis by directly measuring hemoglobin, and a plasma separation unit configured for separating plasma from the blood and arranged so that the separated plasma is supplied to the first sensor structure and the second sensor structure, whereas a rest of the blood is kept apart from the first sensor structure and the second sensor structure, wherein the sensor arrangement is configured as a disposable test strip.

According to another exemplary embodiment of the invention, a monitoring system is provided which comprises a sensor arrangement having the above mentioned properties, and an evaluation unit configured for evaluating sensed information (such as an evaluation of the amount or concentration of the physiological substance, and/or an evaluation of the presence, absence or strength of hemolysis, and/or an evaluation of a degree of impact of detected hemolysis on the accuracy or inaccuracy of a detected amount or concentration of the physiological substance).

The term "physiological subject" or biological object may particularly denote any human being, and any other animal or organism.

The term "physiological substance" may particularly denote any substance which occurs naturally within a physiological subject and is therefore related to the physiology of a living organism, for instance the metabolism, etc. Such a physiological substance may include a biochemically active molecule (such as potassium, glucose), a hormone, a protein, an enzyme, etc.

The term "hemolysis" may particularly denote the rupturing or lysis of red blood cells (which may also be denoted as erythrocytes) and the release of their contents (such as cytoplasm) into surrounding fluid (in particular blood plasma). Hemolysis may occur *in vivo* and/or *in vitro* (i.e. inside and/or outside the body).

The term "detecting the physiological substance is influenceable by hemolysis of the blood" may particularly denote that the chemical and/or physical properties of the physiological substance to be detected and/or its detectability may depend on or may be influenced by the presence or absence and/or the amount of hemolysis of the blood in which the physiological substance to be detected is present.

The term "plasma separation" may particularly denote a process of separating blood (in particular whole blood) into blood plasma and blood cells (in particular only blood cells having a size larger than a predetermined threshold value). In an embodiment, larger blood cells (such as erythrocytes) may be prevented from passing a plasma separation unit, whereas smaller blood cells (such as thrombocytes) may pass the plasma separation unit. In another embodiment, all blood cells (such as erythrocytes and thrombocytes) may be prevented from passing the plasma separation unit. When the plasma separation unit is embodied as a permeable membrane, the molecular weight cut off (MWCO) of such a membrane defines which particles can pass through the membrane, and which not. The molecular weight cut off is correlated with pore sizes of pores in the membrane. Subsequently, selectively the separated plasma may be supplied to a desired location or destination of a sensor arrangement for actual detection.

The term "test strip" may particularly denote a band or piece or strip (for instance of plastic, paper, ceramic, metal and/or other material) used for biological testing. Test strips may be handy mobile laboratories for the semiquantitative detection of ions, organic substances, inorganic substances, etc.

A gist of an exemplary embodiment of the invention is that a first sensor structure sensing a hemolysis-sensitive physiological substance in blood is combined with a second sensor structure sensing hemolysis in the same blood sample. The result of the hemolysis detection can then be used for interpreting the results of the detection of the hemolysis-sensitive physiological substance which can be disturbed by hemolysis. Since the event of hemolysis has the consequence that the drawn blood sample is no longer characteristic for the properties of the blood in the physiological subject (such as a human being), considering hemolysis for determining the physiological substance increases accuracy and reliability of the result. Thus, hemolysis related artefacts in the measurement of the hemolysis-sensitive physiological substance may be efficiently suppressed by considering the reference measurement in form of the hemolysis detection. As a result, the detection of the hemolysis sensitive physiological substance (for instance potassium or an enzyme) is more accurate and more reliable. In addition, the first sensor structure and the second sensor structure may both be configured for electrochemically sensing the physiological substance and the parameter, respectively, so that both sensor structures may share or make use of common equipment, such as a common potentiostat, for the electrochemical sensing. As a result, space consumption may be reduced and the sensor arrangement may thus be provided in a more compact manner.

A further gist of an exemplary embodiment of the invention is that a measurement of a physiological substance and/or of a (physiological) parameter is not carried out on the basis of a whole blood sample, but is carried out selectively on the basis of separated blood plasma only. For that purpose, a plasma separation can be executed before measuring the physiological substance and/or the (physiological) parameter in the separated plasma being substantially free of blood cells. By such a passive plasma separation, a measurement of a physiological substance and/or of a (physiological) parameter in blood can become more accurate, since the measurement of several physiological parameters such as the potassium concentration and/or a parameter indicative of hemolysis can be carried out in a more precise way on the basis of blood plasma rather than on the basis of whole blood.

In the following, further exemplary embodiments of the sensor arrangement, and the monitoring system will be explained.

As mentioned above, the evaluation unit may be configured for evaluating sensed information. In an embodiment, the evaluation unit may determine the information indicative of the physiological substance by evaluating the sensed data captured by the first sensor structure (relating to the sensing of the physiological substance) in view of the sensor data captured by the second sensor structure (relating to the sensing of hemolysis). This may involve a correction of the sensed information concerning the physiological substance in view of the sensed hemolysis. In an embodiment, the evaluation unit may determine the information indicative of the physiological substance and/or of the (physiological) parameter by evaluating the sensed data captured by the sensor structure (relating to the sensing of the physiological substance and/or of the (physiological) parameter) selectively based on the separated plasma portion of the blood. It is also possible that an evaluation unit carries out more than one of the mentioned tasks.

The first sensor structure is configured so that detecting the physiological substance is hemolysis-sensitive. In other words, the result of the detection of the physiological substance by the first sensor structure is influenced by hemolysis of the blood sample under investigation. Thus, additional detection of hemolysis in the same blood sample renders the result of the detection of the physiological substance more reliable.

In an embodiment, the first sensor structure is configured for sensing potassium as the physiological substance. In other words, the first sensor structure may be configured for potassium sensing. Thus, the first sensor structure may determine the potassium ion (i.e. K⁺) concentration in the blood sample, or more preferably in the previously separated plasma of the blood sample only. Since the determination of potassium ion concentration in blood depends sensitively on the fact as to whether the blood sample has suffered hemolysis, an additional determination of a hemolysis related parameter in the blood sample is advantageous for improving accuracy of the potassium determination.

The first sensor structure is configured for electrochemically sensing the physiological substance. Electrochemistry refers to the relationship between electricity, as a measurable and quantitative phenomenon, and identifiable chemical change or properties. In an embodiment, the electricity caused by the physiological substance (such as potassium ions) can be considered as an outcome of a particular chemical property of the blood sample under investigation, i.e. its amount or concentration of the physiological substance. Reactions used during an electrochemical sensing of a physiological substance may involve electric charges moving between electrodes and an electrolyte or ionic species in a solution.

More specifically, the first sensor structure may comprise a reference electrode (in particular a silver/silver chloride (Ag-AgCI) reference electrode) and an ion-sensitive electrode for detecting the physiological substance electrochemically. The reference electrode stays or is kept at a constant electric potential while the ion-sensitive electrode changes its electric potential with the potassium concentration in the sample. The potential difference between the two electrodes may be measured as an electric voltage. This potential difference is indicative of the potassium concentration in the blood. A corresponding first sensor structure with planar electrodes may be manufactured with low effort, for instance by screen printing. Moreover, a corresponding sensor arrangement may be manufactured in a compact flat configuration.

Thus, the first sensor structure (in particular configured for sensing potassium concentration) may be embodied as a potentiometric sensor, i.e. a sensor detecting an electric voltage. As a result, the first sensor structure may be embodied with two electrodes.

The second sensor structure is configured for electrochemically sensing hemolysis (in particular by electrochemically detecting iron). For instance, hemolysis in a blood sample may be detected by using an electrode to measure the reduction of hydrogen peroxide concentration (and/or another oxidizing agent) when hemoglobin in plasma is oxidized or to directly measure hemoglobin, for instance by determining the iron in the hemoglobin. Thus, also the second sensor structure uses an electrochemical measurement principle for determining information about hemolysis.

The second sensor structure is configured for electrochemically sensing hemolysis by directly measuring hemoglobin, for instance by determining the iron in the hemoglobin. This approach might be advantageous over an indirect measurement of hemoglobin, such as by measuring hydrogen peroxide (and/or another oxidizing agent) when hemoglobin in plasma is oxidized. Typically, the hydrogen peroxide measured in an indirect measurement of hemoglobin originates from glucose. Thus, an indirect measurement of hemoglobin by measuring hydrogen peroxide typically requires the additional measurement of the glucose level. By directly measuring hemoglobin, in particular by a direct electrochemical sensing of hemoglobin, the additional measurement (as well as the equipment required for doing so) of the glucose level may be omitted which improves the efficiency and the accuracy of the determination of the hemolysis-indicative parameter as well as the compactness of the sensor arrangement.

More specifically, the second sensor structure may comprise a reference electrode, a working electrode (which may also be denoted as a detection electrode) and a counter electrode. The working electrode may be kept at a constant electric working potential that is used to carry out a redox reaction with the iron ions in hemoglobin. The measured electric current between working electrode and counter electrode is indicative for the concentration of free hemoglobin in the sample. The concentration of free hemoglobin in the sample, in turn, is correlated with the amount of hemolysis of the blood. A corresponding second sensor structure with planar electrodes may be manufactured with low effort, for instance by screen printing. Moreover, a corresponding sensor arrangement may be manufactured in a compact flat configuration. This particularly holds when planar electrodes of the first sensor structure and of the second sensor structures are all formed coplanar, i.e. in a common plane.

Thus, the second sensor structure (in particular configured for sensing hemolysis) may be embodied as an amperometric sensor, i.e. a sensor detecting an electric current. As a result, the second sensor structure may be embodied with three electrodes.

The first sensor structure and the second sensor structure are arranged on and/or integrated in a common test strip. By taking this measure, the detection of the physiological substance and the hemolysis detection are carried out spatially very close together. Therefore, the measurements in terms of the physiological substance and the potential hemolysis are directly comparable with high accuracy. At the same time, a very small amount of blood may be sufficient for carrying out both measurements. A corresponding sensor arrangement is configured as a simply manufacturable test strip which a patient can use at home.

The sensor arrangement is configured as a disposable test strip operable by a patient, even without medical personnel. Due to a hemolysis detection capability integrated on a test strip, undesired hemolysis of a blood sample by improper operation by a patient (who is in many cases a medical laymen) will advantageously not result in a deterioration of the detection of the physiological substance, even when the latter is hemolysis-sensitive. Due to the simple sheet like configuration of the individual constituents of the sensor arrangement and its cost efficient manufacturability by simple processes such as screen printing, the sensor arrangement is also suitable to be used as a single-use or disposable device. A corresponding test strip may be easily operated even by patients without professional medical skills.

In an embodiment, the second sensor structure is configured for sensing hemolysis qualitatively or quantitatively. In other words, the sensor structure may be configured for sensing one of the group consisting of the presence or absence of hemolysis in the blood, and an amount of hemolysis in the blood. A qualitative information about hemolysis may be a simple yes or no result. A quantitative information about hemolysis may be indicative of a value indicating the strength or level of hemolysis. The qualitative or quantitative hemolysis information may be used for interpreting or correcting the results of the hemolysis sensitive detection of the physiological substance.

The sensor arrangement comprises a plasma separation unit configured for separating plasma from or of the blood sample and arranged so that selectively the separated plasma (optionally still comprising small blood cells, but being free of larger blood cells) is supplied to the first sensor structure and/or the second sensor structure. Thus, the plasma separation unit is provided upstream of the first sensor structure and the second sensor structure. By such a plasma separation unit, blood plasma (i.e. water, proteins, ions, optionally small cells, etc.) is separated from cells of the whole blood. Only the plasma is then supplied to the sensor structure(s), whereas a rest of the blood (in particular cells, more particularly large cells) is kept apart from the sensor structure(s). Determination of many physiological substances as well as of hemolysis is carried out with significantly better precision on the basis of plasma than on the basis of whole blood.

In an embodiment, the plasma separation unit may comprise or consist of a filter filtering the blood sample. Such a filter may be embodied as a permeable membrane being permeable only for a part of the constituents of the blood (i.e. may be permeable for the plasma and impermeable for the larger cells of the blood). More specifically, a permeable membrane may be provided as filter having pores which has a smaller diameter on a main surface facing the sensor structure compared to an opposing other main surface facing a user supplying blood to the sensor arrangement. By taking this measure, the active surface of the filter on a side facing the blood sample provided by the user may be enlarged, so that undesired clogging of the filter at this exterior side can be efficiently prevented. For example, the pores may have a size at the surface facing the user's finger larger than essentially 1 µm, particularly in the range between essentially 1 µm and essentially 10 µm. The pores may have a size at the opposing surface facing the sensor structure(s) smaller than essentially 1 µm, particularly in the range between essentially 0.2 µm and essentially 1 µm. Such a membrane may be prepared by extrusion from polysulfone, polyethersulfone or a blend of polyethersulfone and polyvinylpyrrolidone, or on the basis of another polymer. Other possible membrane materials may include inorganic materials like ceramic, glass, stainless steel or derivatized carbon. Undesired clogging of the membrane by large blood cells may be prevented due to the relatively large pore size on the side of the membrane at which the raw blood drop is supplied from the patient. The smaller pores on the opposing other side allow for an efficient transfer of the filtered plasma to the fluidic channel and from there to the one or more sensor structures. However, as an alternative to such a membrane with an anisotropic or inhomogeneous pore structure, it is also possible that a membrane with homogeneous or isotropic pore size over its thickness extension may be used.

In an embodiment, the sensor arrangement comprises a fluidic channel structure, in particular a microfluidic channel structure (i.e. a channel structure having dimensions in the order of micrometers), configured for guiding at least part of the blood (in particular the separated plasma output from the plasma separation unit) to the first sensor structure and the second sensor structure. In such a fluidic channel structure, a capillary or other fluidic conduit may be delimited to precisely define a trajectory along which the blood or part thereof may flow in a defined way (in particular straight to the sensor structure(s)).

Preferably, the guidance of the blood or parts thereof (in particular plasma) along the fluidic channel may be powered or driven by capillary action. More specifically, the sensor arrangement may comprise a sheet with a microfluidic channel configured for guiding the blood or part thereof (in particular the plasma), i.e. the sample fluid, to the sensor structure by capillary action. Capillary action may be denoted as the ability of the blood or part thereof to flow in narrow spaces without the assistance of, or even in opposition to, external forces like gravity. The effect occurs because of intermolecular forces between the blood or part thereof and surrounding solid surfaces delimiting the fluidic channel. If the diameter of the fluidic channel is sufficiently small, then the combination of surface tension (which is caused by cohesion within the blood or part thereof) and adhesive forces between the blood or part thereof and the wall of the fluidic structure act to propel the blood or part thereof. When a microfluidic channel is implemented which generates a capillary force for moving the blood or part thereof to the sensor structure(s), a fluidic pump or another driving unit for driving the sample fluid becomes dispensable. This allows manufacturing the sensor arrangement with low effort and of a test strip type.

As an alternative to a sheet with a fluidic channel, it is also possible to provide a sheet with pronounced liquid absorbing capability, such as a piece of blotting paper.

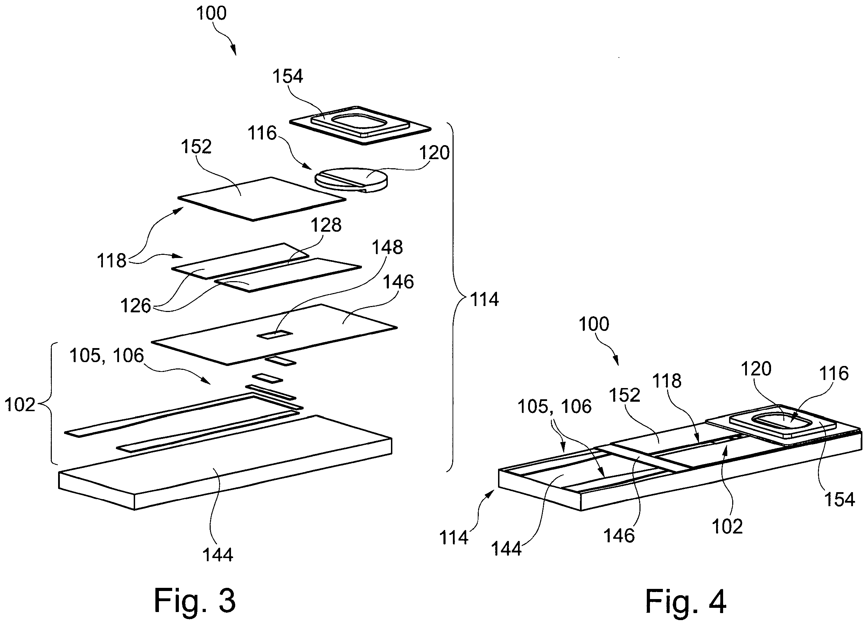

The sensor arrangement as a whole is configured as flat sheet and hence highly compact. The first sensor structure and the second sensor structure may be formed on the basis of flat electrodes structures, preferably all located within a common plane. The plasma separation unit may be a flat filter sheet defined by a porous membrane. The fluidic channel structure may be defined by a recess in a sheet, which recess may be covered by a further sheet so as to fully circumferentially delimit a capillary. When these constituents, are stacked, a sheet type sensor arrangement may be obtained which is configured as a test strip (see Figure 3 and Figure 4).

In an embodiment, the sensor arrangement comprises a semiconductor chip (electronic chip).

In an embodiment, the semiconductor chip may be configured for supporting or assisting the first sensor structure to electrochemically sense the physiological substance and/or the second sensor structure (104) to electrochemically sense the (physiological) parameter. For example, the semiconductor chip may be configured for detecting an electric voltage and/or an electric current. Thus, the semiconductor chip may replace or substitute a potentiostat for the electrochemical sensing.

In addition or alternatively, the semiconductor chip may be configured for transmitting sensed information of the first sensor structure and/or the second sensor structure to an evaluation unit of a monitoring system. By taking this measure, an immediate evaluation of the sensed information may be possible so as to immediately provide physiological results, which may further allow for an immediate reaction or response to the physiological results, such as therapeutic measures depending on the physiological results. In view of the high accuracy and reliability of the sensor arrangement, such immediate reaction to the physiological results may be performed by a patient himself without necessarily requiring a physician. In this regard, the evaluation unit may not only provide the physiological results, but also a proposal for a therapeutic regimen, for instance a proposal for an appropriate adjustment of medication depending on the physiological results.

Preferably, the semiconductor chip is configured for transmitting the sensed information in a wireless and/or contactless manner, in particular via near field communication (NFC). By taking this measure, sensed information of the first sensor structure and/or the second sensor structure to an evaluation unit of a monitoring system may be transmitted in a convenient and hygienic manner.

In an embodiment, the evaluation unit is (or forms) part of a portable electronic device, in particular a smartphone. Hereby, the results of the evaluation, such as the measured physiological results, may be directly displayed by the portable electronic device, for instance a smartphone. Moreover, the evaluation unit may not only provide the physiological results, but also a proposal for a therapeutic regimen, for instance a proposal for an appropriate adjustment of medication depending on the physiological results, such as an adjustment of diuretica medication in case of potassium sensing.

In an embodiment, the evaluation unit is configured for triggering an event depending on the sensed information indicative of hemolysis of the blood. For instance, the triggered event may be a refusal of the detection, a correction of a sensor result depending on sensed hemolysis, and outputting hemolysis information (in particular a corresponding alarm) to a user. Hence, in one embodiment, the patient may be informed (for instance by a display) that the measurement of the physiological substance must be rejected due to the occurrence of measurement disturbing hemolysis. The user may be invited to repeat the measurement. Additionally or alternatively, an algorithm may be carried out correcting the measured value of the physiological substance based on a determined degree of hemolysis. For instance, a lookup table may be used for this purpose from which correction parameters may be retrieved or a (correct, actual) value of the physiological substance may be calculated based on a determined degree of hemolysis. In yet another embodiment, the measurement result may be output to the user but may be accompanied with hemolysis information. For instance, an indication may be given to a user describing the reliability of the measurement results in view of the present hemolysis level. In still other embodiments, a visual, audible and/or haptic alarm may be generated as a warning to a user that the determined value of the physiological substance might lack reliability in view of detected hemolysis.

In an embodiment, the monitoring system comprises an accommodation recess shaped and dimensioned for accommodating at least part of the sensor arrangement so that the sensed information is transmittable from the sensor arrangement to the evaluation unit merely upon inserting the sensor arrangement into the accommodation recess. By such an intuitive insertion operation, a data communication connection may be established between (for instance sensor electrodes of) the test strip type sensor arrangement and (in particular the evaluation unit of) a base station comprising the accommodation recess. The evaluation unit may then process the sensed data to determine a result of the blood test.

The aspects defined above and further aspects of the invention are apparent from the examples of embodiment to be described hereinafter and are explained with reference to these examples of embodiment.

The invention will be described in more detail hereinafter with reference to examples of embodiment but to which the invention is not limited.
Figure 1 shows a monitoring system with a sensor arrangement for monitoring a physiological substance according to an exemplary embodiment of the invention.
Figure 2 shows a detailed view of the sensor arrangement of Figure 1.
Figure 3 shows an exploded view of components of a test strip type sensor arrangement for monitoring a physiological substance in blood of a physiological subject according to an exemplary embodiment of the invention.
Figure 4 shows an assembled view of the sensor arrangement of Figure 3.
Figure 5 shows an assembled view of a sensor arrangement according to an exemplary embodiment of the invention illustrating, in addition to Figure 4, also a second sensor structure and corresponding electrodes.
Figure 6 shows results of the determination of hemolysis by means of a sensor arrangement according to an exemplary embodiment of the invention at various concentrations of hemoglobin.

The illustration in the drawing is schematically. In different drawings, similar or identical elements are provided with the same reference signs.

Before describing further exemplary embodiments in further detail, some basic considerations of the present inventors will be summarized based on which exemplary embodiments have been developed.

According to an exemplary embodiment of the invention, a potassium measurement and a hemolysis measurement are combined in one common test strip. A passive plasma separation is carried out on a microfluidic test strip prior to the determination of a parameter and a physiological substance. Such a sensor arrangement is advantageous for the measurement of potassium and many other parameters (for instance measurement of ammonium, phosphate, lactate dehydrogenase (LDH), phosphatase, triglycerides, creatinine, and bilirubin) which can be measured highly accurately on the basis of plasma.

According to a more specific exemplary embodiment of the invention, a potassium test strip for a patient self-test is provided. Such a test strip is appropriate for carrying out patient self-test concerning potassium ion concentration in a capillary blood sample under consideration of possible artifacts due to hemolysis.

In an exemplary embodiment, a potassium sensor (which may be denoted more generally as first sensor structure) can be applied on a substrate (for instance made of plastic). Such a sensor may be composed of an ion selective electrode (ISE) and an Ag/AgCl reference electrode. Such a sensor arrangement can be manufactured completely by screen printing technique. In particular, screen printing is a highly appropriate manufacturing process for a corresponding electrode structure, a potassium sensitive membrane and a chloride reservoir membrane for the reference electrode. The potassium measurement can be carried out by the patient himself based on a drop of capillary blood from the finger pad. Such a measurement based on a drop of whole blood from the finger pad is conventionally prone for hemolysis (descriptively speaking a non-physiological modification of the blood caused by exerting excessive pressure onto the finger by a patient, i.e. by excessive squeezing). Since hemolysis can distort the measurement of the potassium concentration in the blood, the invention provides an additional hemolysis sensor (which may be denoted more generally as second sensor structure) on the test strip or sensor arrangement.

The hemolysis detection is based on the detection of the redox current of iron in free hemoglobin at a sensor electrode. A passive filter system allows to separate the erythrocytes from the blood sample, since the erythrocytes and other cell constituents of the blood may disturb the hemolysis detection. Hemolysis detection is based, in an embodiment, on the determination of free hemoglobin in blood plasma. The sensor arrangement or test strip advantageously further comprises a filter (which is denoted more generally as plasma separation unit) for separating erythrocytes from the blood sample. Further advantageously, an exemplary embodiment of the invention may further comprise a microfluidic channel (which may be denoted more generally as fluidic channel structure) to guide the separated plasma along the sensor structure(s) in a passive and hemolysis-free way. The result of the hemolysis detection may be used to trigger a predefined event, such as the issuance of a warning to the patient that or when the potassium value is falsified by the occurrence of hemolysis. The result of the hemolysis detection may be further used to correct the determined value of the physiological substance so as to obtain an (at least more) accurate result of the actual value of the physiological substance (i.e. of the value of physiological substance without hemolysis).

Highly advantageously, the described additional hemolysis detection increases the safety during blood potassium determination by a patient and thereby enables the reliable and secure as well as precise self-measurement by a patient on the basis of capillary blood. Conventional test systems without hemolysis detection are allowed only for samples for venous whole blood samples, wherein a blood draw requires the involvement of health personnel.

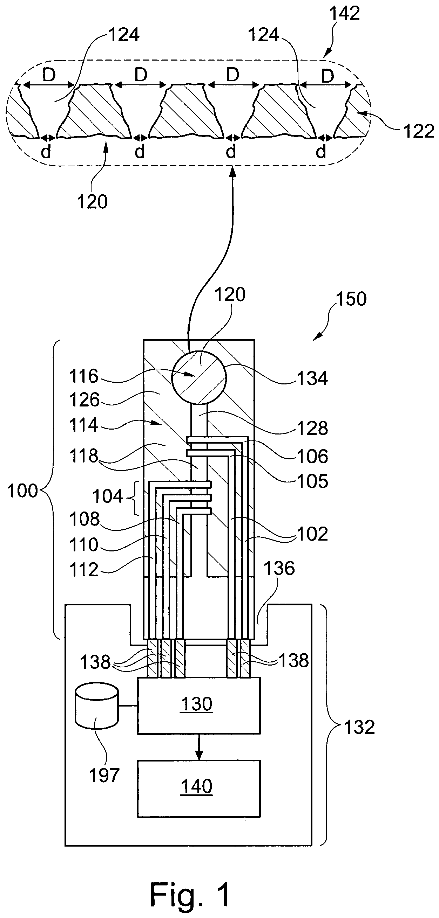

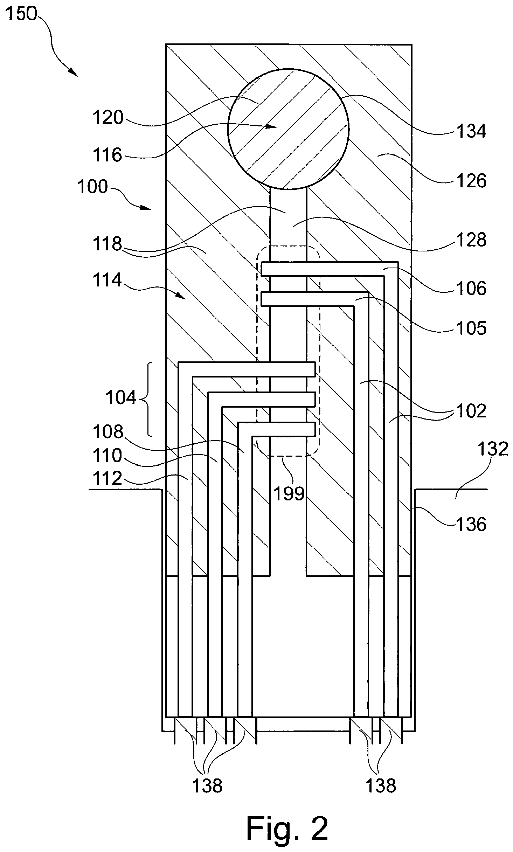

**Figure 1** shows a monitoring system 150 with a sensor arrangement 100 shaped as a flat sheet and configured for monitoring a physiological substance (in the shown embodiment a potassium ion level) in blood of a physiological subject (in the shown embodiment a human being) according to an exemplary embodiment of the invention. **Figure 2** shows a detailed view of the sensor arrangement 100 of Figure 1.

The illustrated monitoring system 150 is composed of the test strip 114 type sensor arrangement 100 and a base station 132 comprising, in turn, an evaluation unit 130 for evaluating sensed information. The mentioned test strip 114 may be a substantially rectangular flat sheet for single use by a patient. More specifically, the sensor arrangement 100 is embodied as a single-use disposable test strip 114 onto which a patient can place a selfdrawn whole blood drop at a blood provision area 134. For the purpose of drawing a drop of whole blood, the patient can puncture his finger at the finger pad, for example using a needle (not shown). For instance, blood sampling may occur by finger pricking. The user may then place this finger on the blood provision area 134 of the sensor arrangement 100 and may apply a slight pressure on the finger pad to guide the drop of whole blood onto the blood provision area 134. Care should be taken to avoid overpressure during this procedure, since this may result in undesired hemolysis of the blood drop. However, when a medical layman, i.e. a patient himself, draws the blood, hemolysis may never be fully excluded in each and every case. Although the event of hemolysis may conventionally disturb the potassium measurement, a provision is taken in the sensor arrangement 100 to avoid false measurement results resulting from hemolysis. This will be described below in further detail.

The electronic base station 132 may be configured as a multiple use medical device (for instance a handheld device) having an accommodation recess 136 shaped and dimensioned for receiving an end portion of the test strip 114 like sensor arrangement 100 before or after the user has drawn a blood drop from his finger and has applied the same to the blood provision area 134. By inserting the sensor arrangement 100 into the accommodation recess 136 of the base station 132 of the monitoring system 150, an electrically conductive connection between electrodes 105, 106, 108, 110, 112 of the sensor arrangement 100 and counter electrodes 138 of the base station 132 is established. As a result, electric potentials present at the electrodes 105, 106 being indicative of the potassium level and the current flow between electrodes 108 and 110 being indicative of the occurrence of hemolysis in the drawn blood drop are supplied to the evaluation unit 134 for evaluation. For example, the evaluation unit 130 may be a processor (for instance a microprocessor or a central processing unit (CPU)) configured for executing machine executable code (for instance a software algorithm) for determining a level of the physiological substance (in the present embodiment the potassium ion level) in the drawn body fluid (in the shown embodiment blood). The evaluation unit 130 may be further configured for determining potential hemolysis in the same blood drop used also for determining the level of the physiological substance.

A result of this analysis may be output via a user interface 140 to a user. The user interface 140 may for example comprise an electronic display for displaying information to a user. Additionally or alternatively, the user interface 140 may also be configured for generating an acoustic output and/or a haptic output, for instance for indicating an alarm (such as a determined degree of hemolysis which renders the determined level of the physiological substance unreliable).

As indicated in Figure 1, a plasma separation unit 116 is provided at the blood provision area 134. The plasma separation unit 116 is configured for separating the supplied whole blood sample into plasma on the one hand and cells on the other hand. Only the separated plasma portion is supplied to the below described sensor structures 102, 104, since the latter are only capable of reliably and precisely determining the respective potassium value and hemolysis level on the basis of plasma rather than whole blood. It is believed that the cells of the blood sample disturb such a measurement. The plasma separation unit 116 is hence configured for separating plasma from the blood and arranged so that only (or substantially only) the separated plasma is supplied to the first sensor structure 102 and the second sensor structure 104.

As can be taken from a detail 142 shown in Figure 1, the plasma separation unit 116 may be embodied as a sheet like filter 120. In the shown embodiment, the sheet like filter 120 has a substantially circular shape so that a patient can put his finger pad directly onto the circular filter 120. As can be taken from the detail 142 showing a schematic cross-section of the filter 120 along a cross sectional plane perpendicular to the paper plane of Figure 1, the filter 120 is formed by a permeable membrane 122 having pores 124. The pores 124 have a smaller diameter, d, on a main surface facing the sensor structures 102, 104 compared to a larger diameter, D, on an opposing other main surface facing a user supplying blood to the sensor arrangement 100. The relatively large pore size on the outer surface of the membrane 122 prevents large blood cells from traversing the membrane 122 and from clogging the pores 124. At the same time, blood plasma (including the small potassium ions to be sensed) is capable of traversing the membrane 122 through the pores 124. The smaller pore size on the interior surface of the membrane 122 facing the sensor structures 102, 104 ensures a proper permeability of the membrane 122 and therefore a rapid and precise detection. In other words, the configuration of the filter 120 ensures quick and substantially complete separation of plasma against cellular components of the blood sample while preventing undesired membrane clogging. Alternatively to the configuration of Figure 1, it is however also possible to implement a filter membrane with homogeneous or isotropic pore dimensions.

As can be taken from Figure 1 as well, the sensor arrangement 100 further comprises a microfluidic channel structure 118 configured for guiding specifically the separated plasma of the drawn whole blood sample to the first sensor structure 102 and the second sensor structure 104 exclusively by capillary action. More specifically, the microfluidic channel structure 118 comprises a flat sheet 126 with a covered recess forming a microfluidic channel 128 configured for guiding exclusively the separated plasma to the sensor structures 102, 104 by capillary action. Thus, the separated plasma is sucked into and along the microfluidic channel 128 without providing a drive mechanism such as a fluidic pump or the like, merely by the impact of capillary forces. This keeps the sensor arrangement 100 compact in size and simple in manufacture.

As can be taken from Figure 1 and Figure 2, the electrodes 105, 106, 108, 110, 112 of the sensor structures 102, 104 partially extend into the microfluidic channel 128 of the microfluidic channel structure 118, so that the separated plasma gets into physically contact with these electrodes 105, 106, 108, 110, 112. In other words, the electrodes 105, 106, 108, 110, 112 cross the microfluidic channel 128 in a crossing region 199 (see Figure 2) so that the electrodes 105, 106, 108, 110, 112 detect electric signals as a result of an electrochemical interaction between the separated blood plasma and the electrodes 105, 106, 108, 110, 112. The electrodes 105, 106, 108, 110, 112 will thus detect electric signals allowing to determine both potassium ion concentration and possible hemolysis in the blood. As a consequence of the described design, the separated plasma may be reliably and precisely conducted to the electrodes 105, 106, 108, 110, 112 for detection without involving the risk of additional hemolysis on the fluidic path from the plasma separation unit 116 to the sensor structures 102, 104.

As an alternative to the microfluidic channel structure 118 designed according to Figure 1, it is possible to provide a sheet of blotting paper in fluid communication with the electrodes 105, 106, 108, 110, 112. The separated plasma may then be soaked by the blotting paper and can thereby be brought in physical contact with the electrodes 105, 106, 108, 110, 112.

For carrying out the actual detection task in terms of potassium ion detection, the sensor arrangement 100 comprises the first sensor structure 102 configured for sensing the physiological substance (potassium in the described embodiment) in the previously separated plasma portion of the blood. Thus, the separated plasma separated by the plasma separation unit 116 is supplied to the sensor structures 102, 104 via the fluidic channel structure 118. In order to measure the potassium ion level electrochemically, the first sensor structure 102 comprises a reference electrode 105 (which is preferably embodied as a silver/silver chloride (Ag/AgCl) reference electrode, and a potassium ion-sensitive electrode 106. The electric voltage between electrodes 105, 106 which is generated in the presence of plasma of the drawn blood drop at the electrodes 105, 106 is a measure for the potassium level in the drawn blood sample. Since the phenomenon of hemolysis may change or influence the potassium concentration in the blood (upon drawing the blood sample), potassium sensing is undesirably influenced by hemolysis of the blood or in other words is hemolysis sensitive. This has the consequence that the measurement of the potassium level in the blood sample of the patient may be falsified when the phenomenon of hemolysis has occurred upon drawing the blood sample by the patient.

In order to overcome the danger of an undesired falsification of the potassium measurement signal in view of the presence of hemolysis, the second sensor structure 104 is provided on the test strip 114 type sensor arrangement 100 and is configured for sensing a hemolysis related parameter in the plasma portion of the drawn blood sample. More specifically, the mentioned additionally measured parameter is indicative of the possible event of hemolysis of the drawn blood sample. The second sensor structure 104 is configured for electrochemically sensing hemolysis in the drawn blood sample. For this purpose, the second sensor structure 104 comprises a reference electrode 108, a detection electrode 110, and a counter electrode 112.

The second sensor structure 104 may be configured for sensing hemolysis either qualitatively (i.e. hemolysis present or not) or quantitatively (i.e. detecting a level or an amount of hemolysis). When detecting hemolysis qualitatively, the presence of hemolysis may be assumed when a detected signal indicative of hemolysis exceeds a predefined threshold value. If the detected signal indicative of hemolysis remains below the predefined threshold value, it can be assumed that no hemolysis (or at least substantially no hemolysis) is present. The mere output of a qualitative indication of the presence (or absence) of hemolysis may be advantageous when a detected level of the physiological substance (such as potassium) is output to the user accompanied by the additional information as to whether the detected signal is reliable in view of the absence of hemolysis or is doubtful as a result of the presence of hemolysis. Such a yes/no logic is a simple guidance for a user as to whether the measurement of the physiological substance is reliable or should be repeated. When detecting hemolysis quantitatively, a gradual value may be output which is indicative of the signal amplitude measured for determining hemolysis. Alternatively, a corresponding one of multiple different hemolysis levels (for example no hemolysis, low hemolysis, moderate hemolysis, high hemolysis, etc.) may be output to the user. The quantitative estimation of hemolysis may also allow to carry out an electronic correction of the measured value of the physiological substance in view of the present intensity of hemolysis.

The first sensor structure 102 and the second sensor structure 104 are integrated in common test strip 114 in the embodiment of Figure 1. As a result, both sensor structures 102, 104 are spatially very close together and get into physically contact with substantially the same blood sample. Consequently, the detection results of both sensor structures 102, 104 are directly comparable to one another and allow a precise consideration of potential hemolysis when interpreting the detected value of the physiological substance, in particular the potassium ion concentration.

As mentioned above, the evaluation unit 130 determines both the level of the physiological substance as well as possible hemolysis based on the sensor signals detected by the electrodes 105, 106, 108, 110, 112. The evaluation unit 130 is further configured for triggering an event depending on the sensed information indicative of hemolysis of the blood. Such a triggered event may be a refusal of the detection, the calculation of a correction of a sensor result depending on sensed hemolysis, and/or the output of hemolysis information to a user. In particular, an optical, acoustical and/or haptic warning or alarm may be output to the user as an indication that hemolysis has been detected and the determined value of the physiological substance might therefore be unprecise or unreliable.

When calculating a correction of the determined value of the physiological substance in view of the detected degree of hemolysis, the evaluation unit 130 may for instance carry out the correction based on correction data which may be stored in a data storage unit 197 (such as a mass storage device, for instance a hard disk) of the base station 132. The evaluation unit 130 may access the data storage unit 197 for reading and/or writing data. For instance, a correction value may be taken from a lookup table (stored in data storage unit 197) correlating a respective amount of hemolysis with a correction parameter by which the determined value of the physiological substance has to be multiplied to obtain a true value of the physiological substance which would be measured in the absence of hemolysis. In a further refined embodiment, the correcting calculation may be performed on the basis of more than one correction parameter. The correction values or parameters may be determined by carrying out reference measurements of the physiological substance at different levels of hemolysis, may be determined on the basis of a theoretical model of hemolysis and its influence of the measurement of the physiological substance, and/or may be determined based on expert knowledge (such as expert rules).

Concluding, Figure 1 and Figure 2 show test strip 114 with a potassium and hemolysis sensing capability. The filter 120 ensures a hemolysis free separation of erythrocytes from the drawn blood sample and ensures that the potassium and hemolysis detection are carried out on the basis of pure plasma. Advantageously, the tiny microfluidic channel 128 transports selectively the separated plasma to the mutually closely arranged electrodes 105, 106, 108, 110, 112 of the sensor structures 102, 104 for electrochemical detection of potassium and hemolysis based on the same sample.

**Figure 3** shows an exploded view of various components of a test strip 114 type sensor arrangement 100 for monitoring a physiological substance in a physiological subject according to an exemplary embodiment of the invention. **Figure 4** shows an assembled view of the sensor arrangement 100 of Figure 3. The second sensor structure 104 and corresponding electrodes 108, 110, 112 are not illustrated in Figure 3 and Figure 4.

As can be taken from Figure 3 and Figure 4, the first sensor structure 102, the plasma separation unit 116, the fluidic channel structure 118, and consequently the sensor arrangement 100 as a whole are manufactured on the basis of stacked flat sheet structures. More specifically, all the mentioned constituents are arranged on a common flat substrate 144. The substrate 144 may be a rectangular sheet of plastic or the like and serves as a support or mounting base for the other constituents of the sensor arrangement 100. The various planar electrodes 105, 106 of the first sensor structure 102 are formed on or attached to the substrate 144. Thereafter, a cover layer 146 with a central through hole 148 may be attached on the substrate 144 covered with the electrodes 105, 106. Specific portions of the electrodes 105, 106 are exposed in an upward direction by the through hole 148. Subsequently, the sheet type microfluidic structure 118 is attached on top of the cover layer 146. More specifically, the sheet 126 is put on the cover layer 146 so that the microfluidic channel 128 is in fluid communication with the through hole 148, and therefore with the exposed portions of the electrodes 105, 106. Subsequently, a lid layer 152 is put on top of the sheet 126 to cover the microfluidic channel 128, thereby delimiting a circumferentially closed microfluidic capillary. The circular filter 120 of the plasma separation unit 116 is assembled so as to be in fluid communication with the microfluidic channel 128. A filter fixation unit 154, which is here embodied as an annular sheet, is put onto the filter 120 for mechanically fixing the latter.

The constituents of the test strip 114 according to Figure 3 and Figure 4 may be manufactured by screen printing.

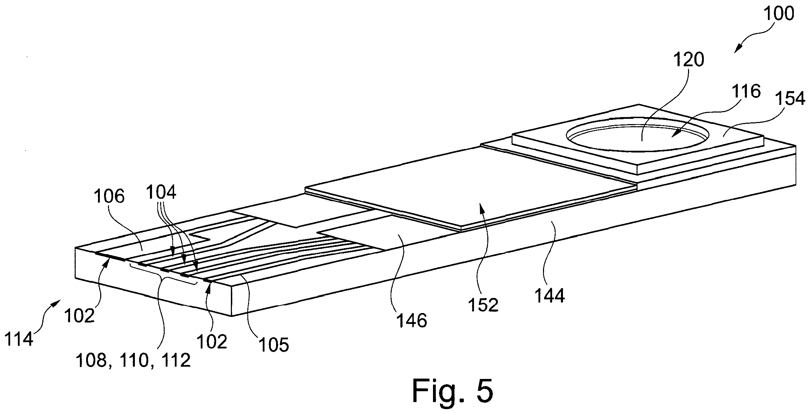

**Figure 5** shows an assembled view of a sensor arrangement 100 according to an exemplary embodiment of the invention illustrating, in addition to the components shown in Figure 3 and Figure 4, also a second sensor structure 104 and corresponding electrodes 108, 110, 112. Also the planar electrodes 108, 110, 112 of the second sensor structure 104 of the test strip 114 according to Figure 5 can be embodied as flat sheets. According to Figure 5, the fluidic channel structure 118 is arranged (for example as a groove) within lid layer 152 and is therefore not visible in Figure 5.

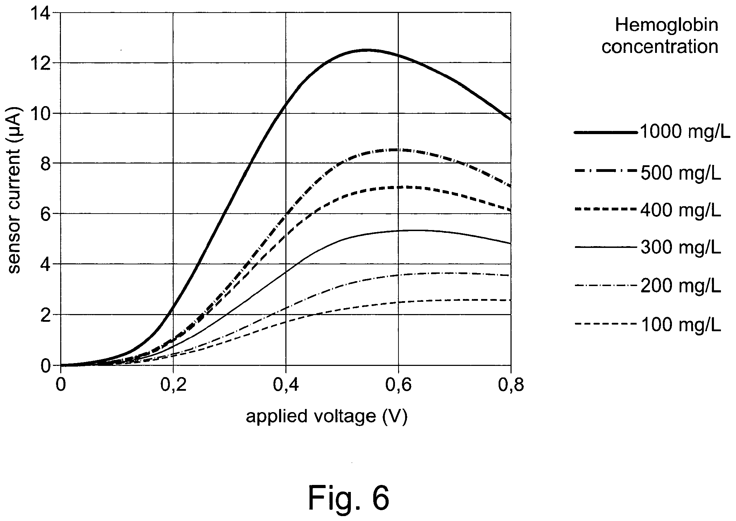

**Figure 6** shows results of the determination of hemolysis by means of a sensor arrangement according to an exemplary embodiment of the invention at various concentrations of hemoglobin. More specifically, hemolysis is measured by direct oxidation of hemoglobin in plasma at the working electrode of the sensor. The sensor signal, measured as sensor current, reaches a maximum at a working potential of 500 mV for a certain hemoglobin concentration. The measured sensor current maximum at 500mV correlates with the hemoglobin concentration in the sample as depicted in Figure 6, and can thus be used to indicate hemolysis in the sample. As it is further evident from Figure 6, the sensor arrangement according to an exemplary embodiment of the invention may operate well over a broad concentration range and may thus be used in a versatile manner for correcting the results of a sensed hemolysis-sensitive physiological substance.

It should be noted that the term "comprising" does not exclude other elements or steps and the "a" or "an" does not exclude a plurality. Also elements described in association with different embodiments may be combined.

It should also be noted that reference signs in the claims shall not be construed as limiting the scope of the claims.

Implementation of the invention is not limited to the preferred embodiments shown in the figures and described above. Instead, a multiplicity of variants are possible which use the solutions shown and the principle according to the invention as defined in the appended claims.

## Claims

1. A sensor arrangement (100) for detecting information indicative of a physiological substance in blood of a physiological subject, the sensor arrangement (100) comprising:
a first sensor structure (102) configured for electrochemically sensing the physiological substance in at least part of the blood, wherein detecting the physiological substance is influenceable by hemolysis of the blood; and
a second sensor structure (104) configured for electrochemically sensing a parameter in at least part of the blood, which parameter is indicative of hemolysis of the blood;
**characterized in that** the sensor arrangement comprises
a plasma separation unit (116) configured for separating plasma from the blood and arranged so that the separated plasma is supplied to the first sensor structure (102) and the second sensor structure (104), whereas a rest of the blood is kept apart from the first sensor structure (102) and the second sensor structure (104),
wherein the second sensor structure (104) is configured for electrochemically sensing hemolysis by directly measuring hemoglobin; and wherein the sensor arrangement (100) is configured as a disposable test strip.

2. The sensor arrangement (100) of claim 1, wherein the first sensor structure (102) is configured for sensing potassium as the physiological substance.

3. The sensor arrangement (100) of claim 1 or 2, wherein the first sensor structure (102) comprises a reference electrode (105), in particular a silver/silver chloride reference electrode, and an ion-sensitive electrode (106).

4. The sensor arrangement (100) of any of claims 1 to 3, wherein the second sensor structure (104) comprises a reference electrode (108), a detection electrode (110) and a counter electrode (112).

5. The sensor arrangement (100) of any of claims 1 to 4, wherein the second sensor structure (104) is configured for sensing hemolysis qualitatively or quantitatively.

6. The sensor arrangement (100) of any of claims 1 to 5, wherein the plasma separation unit (116) comprises or consists of a filter (120), in particular a permeable membrane (122), more particularly a permeable membrane (122) having pores (124) which have smaller diameters (d) on a main surface facing the sensor structure (102) compared to larger diameters (D) on an opposing other main surface facing a user supplying blood to the sensor arrangement (100).

7. The sensor arrangement (100) of any of claims 1 to 6, comprising a fluidic channel structure (118), in particular a microfluidic channel structure, configured for guiding at least part of the blood to the first sensor structure (102) and the second sensor structure (104) by capillary action.

8. The sensor arrangement (100) of any of claims 1 to 7, comprising a semiconductor chip, in particular a semiconductor chip configured for transmitting sensed information of the first sensor structure (102) and/or the second sensor structure (104) to an evaluation unit (130) of a monitoring system (150).

9. The sensor arrangement (100) of claim 8, wherein the semiconductor chip is configured for transmitting the sensed information in a wireless and/or contactless manner, in particular via near field communication (NFC).

10. A monitoring system (150), comprising:
a sensor arrangement (100) of any of claims 1 to 9;
an evaluation unit (130) configured for evaluating sensed information.

11. The monitoring system (150) of claim 10, wherein the evaluation unit (130) is part of a portable electronic device, in particular a smartphone.

12. The monitoring system (150) of claim 10 or 11, wherein the evaluation unit (130) is configured for triggering an event depending on sensed information indicative of hemolysis of the blood, in particular wherein the triggered event comprises at least one of the group consisting of a refusal of the blood test, a correction of a sensor result depending on sensed hemolysis, and an output of hemolysis information, in particular an alarm, to a user.

13. The monitoring system (150) of any of claims 10 to 12, comprising an accommodation recess (136) shaped and dimensioned for accommodating at least part of the sensor arrangement (100) so that the sensed information is transmittable from the sensor arrangement (100) to the evaluation unit (130) upon inserting the sensor arrangement (100) into the accommodation recess (136).

## Patentansprüche

1. Sensoranordnung (100) zum Erfassen von Informationen, die auf eine physiologische Substanz im Blut eines physiologischen Subjekts hinweisen, wobei die Sensoranordnung (100) Folgendes aufweist:
eine erste Sensorstruktur (102), die zum elektrochemischen Erfassen der physiologischen Substanz in mindestens einem Teil des Blutes konfiguriert ist, wobei das Erfassen der physiologischen Substanz durch Hämolyse des Blutes beeinflussbar ist; und
eine zweite Sensorstruktur (104), die zum elektrochemischen Erfassen eines Parameters in zumindest einem Teil des Blutes konfiguriert ist, wobei der Parameter für die Hämolyse des Blutes indikativ ist;
**dadurch gekennzeichnet, dass** die Sensoranordnung Folgendes aufweist
eine Plasmatrenneinheit (116), die zum Abtrennen von Plasma aus dem Blut konfiguriert und so angeordnet ist, dass das abgetrennte Plasma der ersten Sensorstruktur (102) und der zweiten Sensorstruktur (104) zugeführt wird, während ein Rest des Blutes von der ersten Sensorstruktur (102) und der zweiten Sensorstruktur (104) ferngehalten wird, wobei
die zweite Sensorstruktur (104) zum elektrochemischen Erfassen der Hämolyse durch direktes Messen von Hämoglobin konfiguriert ist; und wobei
die Sensoranordnung (100) als Einweg-Teststreifen konfiguriert ist.

2. Sensoranordnung (100) nach Anspruch 1, wobei die erste Sensorstruktur (102) zum Erfassen von Kalium als physiologische Substanz konfiguriert ist.

3. Sensoranordnung (100) nach Anspruch 1 oder 2, wobei die erste Sensorstruktur (102) eine Referenzelektrode (105), insbesondere eine Silber/Silberchlorid-Referenzelektrode, und eine ionensensitive Elektrode (106) umfasst.

4. Sensoranordnung (100) nach einem der Ansprüche 1 bis 3, wobei die zweite Sensorstruktur (104) eine Referenzelektrode (108), eine Detektionselektrode (110) und eine Gegenelektrode (112) aufweist.

5. Sensoranordnung (100) nach einem der Ansprüche 1 bis 4, wobei die zweite Sensorstruktur (104) zur qualitativen oder quantitativen Erfassung der Hämolyse ausgebildet ist.

6. Sensoranordnung (100) nach einem der Ansprüche 1 bis 5, wobei die Plasmatrenneinheit (116) einen Filter (120), insbesondere eine permeable Membran (122), aufweist oder daraus besteht, insbesondere eine permeable Membran (122) mit Poren (124), die auf einer der Sensorstruktur (102) zugewandten Hauptoberfläche kleinere Durchmesser (d) aufweisen verglichen mit größeren Durchmesser (D) auf einer gegenüberliegenden anderen Hauptoberfläche, die einem Benutzer zugewandt ist, der die Sensoranordnung (100) mit Blut versorgt.

7. Sensoranordnung (100) nach einem der Ansprüche 1 bis 6, umfassend eine Fluidkanalstruktur (118), insbesondere eine mikrofluidische Kanalstruktur, die so konfiguriert ist, dass sie zumindest einen Teil des Blutes durch Kapillarwirkung zu der ersten Sensorstruktur (102) und der zweiten Sensorstruktur (104) leitet.

8. Sensoranordnung (100) nach einem der Ansprüche 1 bis 7, umfassend einen Halbleiterchip, insbesondere einen Halbleiterchip, der dazu ausgebildet ist, erfasste Informationen der ersten Sensorstruktur (102) und/oder der zweiten Sensorstruktur (104) an eine Auswerteeinheit (130) eines Überwachungssystems (150) zu übermitteln.

9. Sensoranordnung (100) nach Anspruch 8, wobei der Halbleiterchip dazu ausgebildet ist, die erfassten Informationen drahtlos und/oder berührungslos, insbesondere über Nahfeldkommunikation (NFC), zu übertragen.

10. Überwachungssystem (150), umfassend:
eine Sensoranordnung (100) nach einem der Ansprüche 1 bis 9;
eine Auswerteeinheit (130), die zum Auswerten von erfassten Informationen ausgebildet ist.

11. Überwachungssystem (150) nach Anspruch 10, wobei die Auswerteeinheit (130) Teil eines tragbaren elektronischen Gerätes, insbesondere eines Smartphones, ist.

12. Überwachungssystem (150) nach Anspruch 10 oder 11, wobei die Auswerteeinheit (130) zum Auslösen eines Ereignisses in Abhängigkeit von einer erfassten Information, die auf eine Hämolyse des Blutes hinweist, konfiguriert ist, insbesondere wobei das ausgelöste Ereignis mindestens eines aus der Gruppe aufweist, die aus einer Ablehnung des Bluttests, einer Korrektur eines Sensorergebnisses in Abhängigkeit von einer erfassten Hämolyse und einer Abgabe einer Hämolyse-Information, insbesondere eines Alarms, an einen Benutzer besteht.

13. Überwachungssystem (150) nach einem der Ansprüche 10 bis 12, mit einer Aufnahmevertiefung (136), die zur Aufnahme zumindest eines Teils der Sensoranordnung (100) geformt und dimensioniert ist, so dass die erfasste Information beim Einsetzen der Sensoranordnung (100) in die Aufnahmevertiefung (136) von der Sensoranordnung (100) an die Auswerteeinheit (130) übertragbar ist.

## Revendications

1. Un agencement de capteur (100) pour détecter des informations indicatives d'une substance physiologique dans le sang d'un sujet physiologique, l'agencement de capteur (100) comprenant :
une première structure de capteur (102) configurée pour détecter électrochimiquement la substance physiologique dans au moins une partie du sang, la détection de la substance physiologique pouvant être influencée par l'hémolyse du sang ; et
une deuxième structure de capteur (104) configurée pour détecter électrochimiquement un paramètre dans au moins une partie du sang, lequel paramètre est indicatif d'une hémolyse du sang ;
**caractérisé en ce que** l'agencement de capteur comprend
une unité (116) de séparation de plasma, configurée pour séparer le plasma par rapport au sang et agencée de telle sorte que le plasma séparé soit fourni à la première structure de capteur (102) et à la deuxième structure de capteur (104), tandis qu'un reste du sang est maintenu à l'écart de la première structure de capteur (102) et de la deuxième structure de capteur (104),
la deuxième structure de capteur (104) étant configurée pour détecter électrochimiquement une hémolyse en mesurant directement l'hémoglobine ; et
l'agencement de capteur (100) étant sous la forme d'une bandelette de test jetable.

2. L'agencement de capteur (100) selon la revendication 1, dans lequel la première structure de capteur (102) est configurée pour détecter le potassium en tant que ladite substance physiologique.

3. L'agencement de capteur (100) selon la revendication 1 ou la revendication 2, dans lequel la première structure de capteur (102) comprend une électrode de référence (105), en particulier une électrode de référence argent/chlorure d'argent, et une électrode (106) sensible aux ions.

4. L'agencement de capteur (100) selon l'une quelconque des revendications 1 à 3, dans lequel la deuxième structure de capteur (104) comprend une électrode de référence (108), une électrode de détection (110) et une contre-électrode (112).

5. L'agencement de capteur (100) selon l'une quelconque des revendications 1 à 4, dans lequel la deuxième structure de capteur (104) est configurée pour détecter une hémolyse qualitativement ou quantitativement.

6. L'agencement de capteur (100) selon l'une quelconque des revendications 1 à 5, dans lequel l'unité (116) de séparation de plasma comprend un filtre (120) ou consiste en un tel filtre, en particulier une membrane perméable (122), plus particulièrement une membrane perméable (122) ayant des pores (124) qui ont des diamètres (d) plus petits sur une surface principale faisant face à la structure de capteur (102) par rapport à des diamètres (D) plus grands sur une autre surface principale opposée faisant face à un utilisateur fournissant du sang à l'agencement de capteur (100).

7. L'agencement de capteur (100) selon l'une quelconque des revendications 1 à 6, comprenant une structure de canal fluidique (118), en particulier une structure de canal microfluidique, configurée pour guider au moins une partie du sang vers la première structure de capteur (102) et la deuxième structure de capteur (104) par capillarité.

8. L'agencement de capteur (100) selon l'une quelconque des revendications 1 à 7, comprenant une puce à semi-conducteur, en particulier une puce à semi-conducteur configurée pour transmettre des informations détectées de la première structure de capteur (102) et/ou de la deuxième structure de capteur (104) à une unité d'évaluation (130) d'un système de surveillance (150).

9. L'agencement de capteur (100) selon la revendication 8, dans lequel la puce à semi-conducteur est configurée pour transmettre les informations captées de manière sans fil et/ou sans contact, notamment via une communication en champ proche (NFC).

10. Un système de surveillance (150), comprenant :
un agencement de capteur (100) selon l'une quelconque des revendications 1 à 9 ;
une unité d'évaluation (130) configurée pour évaluer les informations détectées.

11. Le système de surveillance (150) selon la revendication 10, dans lequel l'unité d'évaluation (130) fait partie d'un dispositif électronique portable, notamment un smartphone.

12. Le système de surveillance (150) selon la revendication 10 ou la revendication 11, dans lequel l'unité d'évaluation (130) est configurée pour déclencher un événement en fonction d'informations détectées indiquant une hémolyse du sang, en particulier dans lequel l'événement déclenché comprend au moins un membre du groupe consistant en un refus du test sanguin, une correction d'un résultat de capteur en fonction de l'hémolyse détectée, et une émission d'informations d'hémolyse, notamment une alarme, vers un utilisateur.

13. Le système de surveillance (150) selon l'une quelconque des revendications 10 à 12, comprenant un évidement (136) formant logement, ayant une forme et des dimensions pour loger au moins une partie de l'agencement de capteur (100) de sorte que les informations détectées soient transmissibles de l'agencement de capteur (100) à l'unité d'évaluation (130) lors de l'insertion de l'agencement de capteur (100) dans l'évidement (136) formant logement.
